# EUROPÄISCHE PATENTSCHRIFT

(11) **EP 1 027 064 B1**
(45) Veröffentlichungstag und Bekanntmachung des Hinweises auf die Patenterteilung: **23.03.2005**
(21) Anmeldenummer: 99942693.5
(22) Anmeldetag: 17.09.1999
(51) Int. Cl.: A61K 35/78, A61P 1/00, A61P 15/00

(54) **VERFAHREN ZUR ENTFERNUNG VON UNERWÜNSCHTEN TOXISCHEN ALKALOIDEN AUS PFLANZEN ODER AUS PFLANZLICHEN ZUBEREITUNGEN**
A PROCESS FOR THE REMOVAL OF UNDERSIRED TOXIC ALKALOIDS FROM PLANTS OR FROM VEGETABLE PREPARATIONS
PROCEDE PERMETTANT D'ELIMINER DES PLANTES OU DES PREPARATIONS VEGETALES LES ALCALOIDES TOXIQUES INDESIRABLES

(30) Priorität: 17.09.1998 CH 189698
(43) Veröffentlichungstag der Anmeldung: 16.08.2000
(73) Patentinhaber: Frutarom Schweiz AG, 8820 Wädenswil (CH)
(72) Erfinder: STEINBACH, Thomas, D-58339 Breckerfeld (DE); KREUTER, Matthias-Heinrich, CH-8880 Walenstadt (CH); TITTEL, Gerolf, Heribert, Hugo, Martin, D-82166 Gräfelfing (DE)
(74) Vertreter: Zink, Markus Peter, Dr.
(86) Internationale Anmeldenummer: PCT/CH1999/000446
(87) Internationale Veröffentlichungsnummer: WO 2000/016789

(56) Entgegenhaltungen:
- EP-A- 0 441 672
- EP-A- 0 730 830
- DE-A- 2 755 577
- DE-B- 1 289 400
- US-A- 4 767 861

## Beschreibung

Die vorliegende Erfindung betrifft ein Verfahren zur Entfernung von unerwünschten, toxischen Alkaloiden, einschliesslich die Vorstufen und die Folgeprodukte dieser Verbindungen, aus Pflanzen oder aus pflanzlichen Zubereitungen.

Die vorliegende Erfindung betrifft auch pflanzliche Zubereitungen, welche frei oder nahezu frei sind von unerwünschten, toxischen Alkaloiden, einschliesslich die Vorstufen und die Folgeprodukte dieser Verbindungen.

Die vorliegende Erfindung betrifft ferner ein Medikament, das eine pflanzliche Zubereitung enthält, welche frei oder nahezu frei ist von unerwünschten, toxischen Alkaloiden, einschliesslich die Vorstufen und die Folgeprodukte dieser Verbindungen.

Zahlreiche Pflanzen und daraus gewonnene pflanzliche Zubereitungen enthalten unerwünschte, toxische Alkaloide, insbesondere solche Alkaloide, welche ein quaternäres Stickstoffatom mit einer konjugierten aromatischen Ringstruktur und/oder konjugierten Doppelbindungen enthalten und zu Adduktbildungen mit anderen Molekülen fähig sind.

Ebenso findet man in diesen Pflanzen und den daraus gewonnenen pflanzlichen Zubereitungen Vorstufen dieser toxischen Alkaloide, die durch äussere Einwirkungen zu den toxischen Alkaloiden umgewandelt werden können.

Auch Folgeprodukte dieser Verbindungen können toxisch sein.

Beispiele solcher unerwünschter Verbindungen sind Benzyl-, Isochinolin-, Phenanthren- und Benzophenanthridin-Alkaloide, sofern sie ein quaternäres Stickstoffatom mit einer konjugierten aromatischen Ringstruktur und/oder konjugierten Doppelbindungen enthalten und zu Adduktbildungen mit anderen Molekülen fähig sind.

Die Ursache für die unerwünschten Eigenschaften liegt auch im amphoteren Charakter dieser Verbindungen, welcher für zellmembrantoxische Wirkungen verantwortlich ist.

Zwei typische Vertreter dieser Strukturmerkmale sind Sanguinarin und Chelerythrin, welche praktisch in der gesamten Familie der Papaveraceen vorkommen.

Sanguinarin und Chelerythrin zerstören bereits in der jeweiligen Pflanze beim Austritt aus Vakuolen die Tonoplasten-Membran (Lysis).

Wenn Sanguinarin und Chelerythrin mit menschlichen Hautzellen in Kontakt kommen, führt dies zu Reizungen.

Für Medikamente, welche pflanzliche Zubereitungen mit den oben genannten Verbindungen enthalten, sind hepatotoxische Nebenwirkungen beschrieben worden; siehe Arzneimitteltelegramm, 11, 1997.

Hierbei handelte es sich um Zubereitungen aus Chelidonium majus L., die neben den membrantoxischen Verbindungen Sanguinarin und Chelerythrin in sehr grossen Mengen deren Vorstufen enthalten.

Diese Vorstufen bilden unter äusseren Einwirkungen - beispielsweise Einfluss von Säure, Oxidationen, etc. - Sanguinarin und Chelerythrin.

Die Verbindungen Sanguinarin und Chelerythrin sind hepatotoxisch und töten bereits in geringen Konzentrationen (Mikromol) Hepatozyten in vitro ab.

Der Einsatz solcher pflanzlicher Zubereitungen beinhaltet daher sehr grosse Risiken, da der arzneilichen Wirkung (Nutzen) schwere Nebenwirkungen (Risiko) entgegenstehen.

In US PS 4 767 861 wird ein Verfahren zur Extraktion und Gewinnung von Benzo-c-phenanthridin-Alkaloiden aus Pflanzen der Familien der Papaveraceen, Fumariaceen und Berberidaceen beschrieben.

Dieses Verfahren beinhaltet folgende wesentliche Verfahrensschritte:
a) Extraktion der Alkaloide mit Methanol und Überführung der Alkaloide durch Säurezugabe in ihre Salze,
b) Gewinnung dieser angesäuerten methanolischen Lösung,
c) Ausfällung der Alkaloide als Zink-Salze,
d) Trocknung.

Der so gewonnene getrocknete Festkörper wird in einer verdünnten Zitronensäurelösung suspendiert. Diese Suspension wird erhitzt und heiss filtriert.

Durch die Hinzugabe von NaCl wird erneut ein Festkörper erhalten, welcher gewonnen wird.

Mit dem in EP 0 730 830 A1 beschriebenen Verfahren werden ausschliesslich Pestizide, Pflanzenschutzmittel, Schädlingsbekämpfungsmittel, insbesondere Fungizide, Insektizide, Akarizide, Nematizide, Herbizide, oder Umweltgifte, wie polyhalogenierte, insbesondere polychlorierte, Biphenyle, Dioxine, oder organische Lösungsmittel, wie Benzol, Chloroform, Tetrachlorkohlenstoff, oder Syntheserückstände, wie Alkylhalogenide, halogenierte Aromaten und Heteroaromaten, wie Chlorpyridine aus Getränken oder pflanzlichen Zubereitungen entfernt.

Es ist ein Ziel der vorliegenden Erfindung, pflanzliche Zubereitungen zur Verfügung zu stellen, welche frei oder nahezu frei sind von unerwünschten, toxischen Alkaloiden, einschliesslich die Vorstufen und die Folgeprodukte dieser Verbindungen.

Diese pflanzlichen Zubereitungen sollen die arzneiliche Wirkung aufweisen aber frei oder nahezu frei von hepatotoxischen Nebenwirkungen sein.

Es ist ein weiteres Ziel der vorliegenden Erfindung, Verfahren zur Verfügung zu stellen, mit denen diese pflanzlichen Zubereitungen hergestellt werden können.

Es soll auch ein Medikament zur Verfügung gestellt werden, welches eine pflanzliche Zubereitung enthält, welche frei oder nahezu frei ist von unerwünschten, toxischen Alkaloiden, einschliesslich die Vorstufen und die Folgeprodukte dieser Verbindungen.

Dieses Medikament soll die arzneiliche Wirkung aufweisen aber frei oder nahezu frei von hepatotoxischen Nebenwirkungen sein.

Die obigen Ziele werden mit den erfindungsgemässen Verfahren, den erfindungsgemässen pflanzlichen Zubereitungen und den erfindungsgemässen Medikamenten erreicht.

Das erfindungsgemässe Verfahren zur Entfernung von unerwünschten, toxischen Alkaloiden, einschliesslich die Vorstufen und die Folgeprodukte dieser Verbindungen,
wobei diese unerwünschten Verbindungen ein quaternäres Stickstoffatom mit einer konjugierten aromatischen Ringstruktur und/oder konjugierten Doppelbindungen enthalten und zu Adduktbildungen mit anderen Molekülen fähig sind, insbesondere Benzyl-, Isochinolin-, Phenanthren- und Benzophenanthridin-Alkaloide mit diesen Strukturmerkmalen, einschliesslich beliebige Gemische davon, wobei Berberin und Coptisin ausgenommen sind,
aus Pflanzen oder aus pflanzlichen Zubereitungen, ist dadurch gekennzeichnet, dass man
- in einem ersten Schritt die Pflanze oder die pflanzliche Zubereitung, welche die genannten unerwünschten Verbindungen enthält, mit einer solchen lipophilen Phase in Kontakt bringt oder vermischt, dass sich die zu entfernenden, unerwünschten Verbindungen in dieser lipophilen Phase lösen oder daran adsorbiert werden und sich hierin quantitativ oder nahezu quantitativ anreichern,
wobei man im Falle einer pflanzlichen Zubereitung diese vorgängig auf einen Feststoffgehalt von 1 Gew.-% bis 20 Gew.-% durch die Hinzugabe von Wasser oder eines beliebigen Gemisches von Wasser und einem C₁- bis C₄- Alkohol, einem C₁- bis C₅-Keton, einem C₁- bis C₅- Aldehyd einstellt, bezogen auf die so erhaltene Gesamtmischung,
- in einem zweiten Schritt die lipophile Phase, welche nun die unerwünschten Verbindungen enthält, von der entsprechenden Pflanze oder von der entsprechenden pflanzlichen Zubereitung abtrennt, und
- in einem dritten Schritt die so gereinigte Pflanze oder die so gereinigte pflanzliche Zubereitung gewinnt.

Die erfindungsgemässen pflanzlichen Zubereitungen sind frei oder nahezu frei von unerwünschten, toxischen Alkaloiden, einschliesslich die Vorstufen und die Folgeprodukte dieser Verbindungen, werden gemäss dem erfindungsgemässen Verfahren hergestellt, und enthalten Teil- oder Vollextrakte aus Heil- und/oder Gewürzpflanzen oder Teilen davon, ausgewählt aus der Gruppe, bestehend aus
Berberis vulgaris L.
Peumus boldus Mol.
Sanguinaria canadensis L.
Chelidonium majus L.
Fumaria officinalis L.
Eschscholzia californica Cham.
Papaver somniferum L.
Papaver bracteatum Lindl.
Papaver rhoeas L..

Das erfindungsgemässe Medikament ist dadurch gekennzeichnet, dass es eine pflanzliche Zubereitung enthält, welche frei oder nahezu frei ist von unerwünschten, toxischen Alkaloiden, einschliesslich die Vorstufen und die Folgeprodukte dieser Verbindungen, und dass es zur Verhinderung, zur Behandlung oder zur Nachbehandlung von
- krampfartigen Beschwerden im Bereich der Gallenwege und des Magen-Darmtraktes,
- Menstruationsbeschwerden,
- Zahn- und Kieferschmerzen,
- Ulzerationen des Magen-Darmtraktes,
- Colitis ulcerosa und Morbus Crohn,
- hypertrophen Hautveränderungen,
- Psoriasis vulgaris und Neurodermitis,
- Warzen verschiedenster Genese,
- Cholecystitis, Cholelithiasis, Ikterus catarrhalis, Enteritiden, Ikterus infektiosus und latenten Hepatopathien, insbesondere Hepatitiden,
- dyspeptische Oberbauchbeschwerden, insbesondere bei funktionellen Beschwerden des ableitenden Gallensystems,
dient,
wobei die pflanzliche Zubereitung gemäss dem erfindungsgemässen Verfahren hergestellt ist, und
wobei die pflanzliche Zubereitung ein Teil- oder Vollextrakt aus Heil- und/oder Gewürzpflanzen oder Teilen davon ist, ausgewählt aus der Gruppe, bestehend aus
Berberis vulgaris L.
Peumus boldus Mol.
Sanguinaria canadensis L.
Chelidonium majus L.
Fumaria officinalis L.
Eschscholzia californica Cham.
Papaver somniferum L.
Papaver bracteatum Lindl.
Papaver rhoeas L..

Die bevorzugten erfindungsgemässen pflanzlichen Zubereitungen, enthaltend einen Teil- oder Vollextrakt aus der Pflanze Chelidonium majus L., insbesondere Herba Chelidonii, welche frei oder nahezu frei sind von unerwünschten, toxischen Alkaloiden, einschliesslich die Vorstufen und die Folgeprodukte dieser Verbindungen, wobei die unerwünschten, toxischen Alkaloide Sanguinarin und Chelerythrin sind, einschliesslich beliebige Gemische davon, sind dadurch erhältlich, dass man
a) die entsprechende Zubereitung auf einen Feststoffgehalt von 1 Gew.-% bis 20 Gew.-% durch die Hinzugabe von Wasser oder eines beliebigen Gemisches von Wasser und einem C₁- bis C₄-Alkohol, einem C₁- bis C₅-Keton, einem C₁- bis C₅-Aldehyd, einstellt, bezogen auf die so erhaltene Gesamtmischung, und mit einer solchen lipophilen Phase in Kontakt bringt oder vermischt, dass sich die zu entfernenden, unerwünschten Verbindungen in dieser lipophilen Phase lösen oder daran adsorbiert werden und sich hierin quantitativ oder nahezu quantitativ anreichern,
b) die erhaltene Mischung auf eine Temperatur im Bereich von -30°C bis +30°C bringt und dabei die unerwünschten Verbindungen von einem flüssigen Aggregatzustand in einen festen Aggregatzustand, unter Verwendung von Fetten, Wachsen oder Paraffinen, überführt,
c) die festen, unerwünschten Verbindungen mittels Membran-Separation abtrennt, und
d) die so gereinigte pflanzliche Zubereitung gewinnt.

Bevorzugte Ausführungsformen dieser Erfindungsgegenstände sind in den abhängigen Ansprüchen definiert.

Wenn bei der Gewinnung der pflanzlichen Zubereitung im dritten Schritt im erfindungsgemässen Verfahren Folgeprodukte gebildet werden, dann wird die Bildung dieser Folgeprodukte vorzugsweise gemäss dem in EP 0 826 372 A2 beschriebenen Verfahren verhindert.

Die nachfolgenden Beispiele sollen die vorliegende Erfindung illustrieren.

### Beispiel 1

a.) 1 kg Herba Chelidonii (Chelidonium majus L.) wurde während 3 Stunden mit 70 % m/m Ethanol im Verhältnis 1:10 bei einer Temperatur von 40°C bis 50°C extrahiert.
   Anschliessend wurde filtriert.
   Das erhaltene Filtrat wurde unter reduziertem Druck (50 - 200 mbar) und bei einer Temperatur von 40°C bis 60°C auf einen Feststoffgehalt von 20 % m/m eingeengt.
b.) Dieser Ansatz wurde unter Rühren mit einer Mischung aus Wasser und Ethanol auf einen Gehalt an Feststoffen von 10 % m/m und einer Ethanolkonzentration von 30 % m/m eingestellt.
   Dieses Gemisch wurde anschliessend mit geschmolzenem Kokosfett (50°C) versetzt, bis ein Kokosfettgehalt von 10 % m/m, bezogen auf das erhaltene Gemisch, resultierte.
c.) Dieses Gemisch wurde bei einer Temperatur von 22°C bis 25°C während 30 Minuten intensiv gerührt und anschliessend über eine konditionierte Cross Flow Membran (10 Minuten, 30 % m/m Ethanol als Konditionierungsmittel, Porenweite 0,2 Mikrometer) ultrafiltriert.
d.) Das so erhaltene Filtrat wurde mit 15 % m/m, bezogen auf den Feststoffgehalt, eines Polyvinylpyrrolidons mit einem mittleren Molekulargewicht von 90000 (Kollidon 90 der Firma BASF) versetzt und bei einer Temperatur von 40°C bis 60°C unter reduziertem Druck (50-200 mbar) auf einen Feststoffgehalt von 55 % m/m eingeengt.

Nach Zusatz von 10 % m/m gefällter Kieselsäure, bezogen auf den Feststoffgehalt, wurde bei einer Temperatur von 30°C bis 60°C unter reduziertem Druck (150 bis 20 mbar) getrocknet; siehe EP 0 826 372 A2.

Man erhielt 200 g eines Trockenextraktes mit einem Alkaloidgehalt von 3,4 % m/m gemäss Deutschem Arzneibuch (DAB).

Der Gehalt an Sanguinarin, Chelerythrin, Dihydrosanguinarin und 2 weiterer Vorstufen betrug insgesamt weniger als 5 ppm (HPLC).

### Beispiel 2

### Analog Beispiel 1 aber unter Auslassung der Reinigungsschritte b.) und c.).

Man erhielt 220 g eines Trockenextraktes mit einem Alkaloidgehalt von 3,6 % m/m gemäss DAB.

Der Gehalt an Sanguinarin, Chelerythrin, Dihydrosanguinarin und 2 weiterer Vorstufen betrug insgesamt 300 ppm (HPLC).

Eine vergleichende chromatographische Fingerprint-Analyse (HPLC) der gemäss Beispiel 1 und Beispiel 2 erhaltenen Trockenextrakte zeigte in der dominierenden Fraktion der gewünschten Alkaloide Stylopin, Coptisin, Berberin, Chelidonin, Protopin und Allocryptopin keine qualitativen und keine quantitativen Unterschiede.

Die Fraktion der unerwünschten Alkaloide Sanguinarin, Chelerythrin, Dihydrosanguinarin und 2 weiterer Vorstufen war im Trockenextrakt gemäss Beispiel 1 praktisch nicht vorhanden, währenddem im Trockenextrakt gemäss Beispiel 2 das für Chelidonium majus L. typische Substanzspektrum vorhanden war.

## Patentansprüche

1. Verfahren zur Entfernung von unerwünschten, toxischen Alkaloiden, einschliesslich die Vorstufen und die Folgeprodukte dieser Verbindungen,
wobei diese unerwünschten Verbindungen ein quaternäres Stickstoffatom mit einer konjugierten aromatischen Ringstruktur und/oder konjugierten Doppelbindungen enthalten und zu Adduktbildungen mit anderen Molekülen fähig sind, insbesondere Benzyl-, Isochinolin-, Phenanthren- und Benzophenanthridin-Alkaloide mit diesen Strukturmerkmalen, einschliesslich beliebige Gemische davon, wobei Berberin und Coptisin ausgenommen sind,
aus Pflanzen oder aus pflanzlichen Zubereitungen, **dadurch gekennzeichnet, dass** man
- in einem ersten Schritt die Pflanze oder die pflanzliche Zubereitung, welche die genannten unerwünschten Verbindungen enthält, mit einer solchen lipophilen Phase in Kontakt bringt oder vermischt, dass sich die zu entfernenden, unerwünschten Verbindungen in dieser lipophilen Phase lösen oder daran adsorbiert werden und sich hierin quantitativ oder nahezu quantitativ anreichern,
wobei man im Falle einer pflanzlichen Zubereitung diese vorgängig auf einen Feststoffgehalt von 1 Gew.-% bis 20 Gew.-% durch die Hinzugabe von Wasser oder eines beliebigen Gemisches von Wasser und einem C₁- bis C₄- Alkohol, einem C₁- bis C₅-Keton, einem C₁- bis C₅- Aldehyd einstellt, bezogen auf die so erhaltene Gesamtmischung,
- in einem zweiten Schritt die lipophile Phase, welche nun die unerwünschten Verbindungen enthält, von der entsprechenden Pflanze oder von der entsprechenden pflanzlichen Zubereitung abtrennt, und
- in einem dritten Schritt die so gereinigte Pflanze oder die so gereinigte pflanzliche Zubereitung gewinnt.

2. Verfahren nach Anspruch 1, **dadurch gekennzeichnet, dass** die Vorstufen der unerwünschten Verbindungen Dihydrosanguinarin und Dihydrochelerythrin sind, einschliesslich beliebige Gemische davon.

3. Verfahren nach einem der Ansprüche 1 bis 2, **dadurch gekennzeichnet, dass** die toxischen Alkaloide Sanguinarin und Chelerythrin sind, einschliesslich beliebige Gemische davon.

4. Verfahren nach einem der Ansprüche 1 bis 3, **dadurch gekennzeichnet, dass** die pflanzlichen Zubereitungen Aufgüsse, Tinkturen, Fluida, Spissum-Extrakte, Siccum-Extrakte, Tropflösungen, Säfte, Tonika, einspritzbare und/oder injizierbare Zubereitungen sind.

5. Verfahren nach einem der Ansprüche 1 bis 4, **dadurch gekennzeichnet, dass** die pflanzlichen Zubereitungen Teil- oder Vollextrakte aus Heil- und/oder Gewürzpflanzen oder Teilen davon sind, insbesondere ausgewählt aus der Gruppe, bestehend aus
Berberis vulgaris L.
Peumus boldus Mol.
Sanguinaria canadensis L.
Chelidonium majus L.
Fumaria officinalis L.
Eschscholzia californica Cham.
Papaver somniferum L.
Papaver bracteatum Lindl.
Papaver rhoeas L..

6. Verfahren nach einem der Ansprüche 1 bis 5, **dadurch gekennzeichnet, dass** die lipophile Phase tierischen, pflanzlichen, mineralischen oder synthetischen Ursprungs ist, und insbesondere nicht toxisch, nicht leicht entzündlich, nicht explosiv und nicht flüchtig ist, und vorzugsweise ausgewählt ist aus
- Fetten, wie Kakaobutter, Kokosfett;
- Ölen, wie Neutralöle, Sonnenblumenöl, fraktioniertes Kokosnussöl, wie Miglyol;
- Wachsen, wie Stearine, Yoyobaöl, Bienenwachs, Walrat, Carnaubawachs;
- Paraffinen, einschliesslich Vaseline;
- Lipoiden;
- Sterinen; und
- Kunstharzen, insbesondere Polystyrolharze,
wobei alle diese Verbindungen, einzeln oder als Gemisch, vorzugsweise die Erfordernisse/Definitionen im Deutschen Arzneibuch, DAB, oder in der Britischen Pharmacopoe, BP, oder gemäss dem Food Chemical Codex, FCC, in den Vereinigten Staaten von Amerika erfüllen, respektive diesen Erfordernissen/Definitionen entsprechen müssen.

7. Verfahren nach einem der Ansprüche 1 bis 6, **dadurch gekennzeichnet, dass** im ersten Schritt die Vermischung der Komponenten bei einer solchen Temperatur erfolgt, welche zwischen dem Gefrierpunkt und dem Siedepunkt des jeweiligen Gemisches liegt, wobei eine Temperatur im Bereich von Raumtemperatur bis 70°C bevorzugt ist.

8. Verfahren nach einem der Ansprüche 1 bis 7, **dadurch gekennzeichnet, dass** man im ersten Schritt die Komponenten während etwa 1 Stunde miteinander vermischt, insbesondere durch Schütteln oder Rühren.

9. Verfahren nach einem der Ansprüche 1 bis 8, **dadurch gekennzeichnet, dass** im zweiten Schritt die Abtrennung entweder mittels einer Phasentrennung von 2 flüssigen Phasen oder mittels einer Phasentrennung einer flüssigen und einer festen Phase erfolgt.

10. Verfahren nach einem der Ansprüche 1 bis 9, **dadurch gekennzeichnet, dass** im zweiten Schritt die Abtrennung von 2 flüssigen Phasen mittels Membran-Separation erfolgt, wobei Membranen mit Porenöffnungen im Bereich von 0,001 bis 1,0 Mikrometer, insbesondere von 0,1 bis 0,3 Mikrometer, bevorzugt sind, insbesondere Glas-, Metall-, Keramik- und Kunststoffmembranen, beispielsweise aus Polypropylen oder Teflon.

11. Verfahren nach einem der Ansprüche 1 bis 10, **dadurch gekennzeichnet, dass** man im Falle von pflanzlichen Zubereitungen
- im zweiten Schritt die erhaltene Mischung auf eine Temperatur im Bereich von -30°C bis +30°C bringt und dabei die unerwünschten Verbindungen von einem flüssigen Aggregatzustand in einen festen Aggregatzustand überführt,
- im dritten Schritt die festen, unerwünschten Verbindungen abtrennt, und
- im vierten Schritt die so gereinigte pflanzliche Zubereitung gewinnt.

12. Pflanzliche Zubereitungen, welche frei oder nahezu frei sind von unerwünschten, toxischen Alkaloiden, einschliesslich die Vorstufen und die Folgeprodukte dieser Verbindungen,
wobei die pflanzlichen Zubereitungen gemäss dem Verfahren nach einem der Ansprüche 1 bis 11 hergestellt sind, und
wobei die pflanzlichen Zubereitungen Teiloder Vollextrakte aus Heil- und/oder Gewürzpflanzen oder Teilen davon sind, ausgewählt aus der Gruppe, bestehend aus
Berberis vulgaris L.
Peumus boldus Mol.
Sanguinaria canadensis L.
Chelidonium majus L.
Fumaria officinalis L.
Eschscholzia californica Cham.
Papaver somniferum L.
Papaver bracteatum Lindl.
Papaver rhoeas L..

13. Medikament, **dadurch gekennzeichnet, dass** es eine pflanzliche Zubereitung enthält, welche frei oder nahezu frei ist von unerwünschten, toxischen Alkaloiden, einschliesslich die Vorstufen und die Folgeprodukte dieser Verbindungen, und dass es zur Verhinderung, zur Behandlung oder zur Nachbehandlung von
- krampfartigen Beschwerden im Bereich der Gallenwege und des Magen-Darmtraktes,
- Menstruationsbeschwerden,
- Zahn- und Kieferschmerzen,
- Ulzerationen des Magen-Darmtraktes,
- Colitis ulcerosa und Morbus Crohn,
- hypertrophen Hautveränderungen,
- Psoriasis vulgaris und Neurodermitis,
- Warzen verschiedenster Genese,
- Cholecystitis, Cholelithiasis, Ikterus catarrhalis, Enteritiden, Ikterus infektiosus und latenten Hepatopathien, insbesondere Hepatitiden,
- dyspeptische Oberbauchbeschwerden, insbesondere bei funktionellen Beschwerden des ableitenden Gallensystems,
dient,
wobei die pflanzliche Zubereitung gemäss dem Verfahren nach einem der Ansprüche 1 bis 11 hergestellt ist, und
wobei die pflanzliche Zubereitung ein Teil- oder Vollextrakt aus Heil- und/oder Gewürzpflanzen oder Teilen davon ist, ausgewählt aus der Gruppe, bestehend aus
Berberis vulgaris L.
Peumus boldus Mol.
Sanguinaria canadensis L.
Chelidonium majus L.
Fumaria officinalis L.
Eschscholzia californica Cham.
Papaver somniferum L.
Papaver bracteatum Lindl.
Papaver rhoeas L..

14. Medikament nach Anspruch 13, **dadurch gekennzeichnet, dass** es in einer pharmazeutisch akzeptablen Darreichungsform vorliegt, insbesondere in
- festen Darreichungsformen zur oralen Applikation, insbesondere in der Form einer Tablette, einer Filmtablette, eines Dragees, eines Pellets, einer Hartgelatine-Kapsel, einer Weichgelatine-Kapsel,
- flüssigen Darreichungsformen zur oralen, parenteralen, rektalen, vaginalen und topischen Applikation, insbesondere in der Form einer Tropflösung, eines Sprays, einer Injektionslösung, eines Sirups,
- halbfesten Darreichungsformen zur topischen, oralen, rektalen und vaginalen Applikation, insbesondere in der Form einer Crème, eines Gels, einer Salbe, eines Pflasters, einer Paste, eines Suppositoriums.

15. Medikament nach einem der Ansprüche 13 bis 14, **dadurch gekennzeichnet, dass** die pflanzliche Zubereitung in Nano-Kapseln oder in Liposomen eingearbeitet ist.

16. Medikament nach einem der Ansprüche 13 bis 15, **dadurch gekennzeichnet, dass** es noch wenigstens einen Zusatz- und/oder Hilfs- und/oder Wirkstoff enthält.

17. Medikament nach einem der Ansprüche 13 bis 16, **dadurch gekennzeichnet, dass** die pflanzliche Zubereitung von 0,1 Gew.-% bis 10 Gew.-%, vorzugsweise von 1,0 Gew.-% bis 6,0 Gew.-%, Gesamtalkaloide enthält, bezogen auf die pflanzliche Zubereitung.

18. Verwendung einer pflanzlichen Zubereitung, welche frei oder nahezu frei ist von unerwünschten, toxischen Alkaloiden, einschliesslich die Vorstufen und die Folgeprodukte dieser Verbindungen, zur Herstellung eines Medikamentes zur Verhinderung, zur Behandlung oder zur Nachbehandlung von
- krampfartigen Beschwerden im Bereich der Gallenwege und des Magen-Darmtraktes,
- Menstruationsbeschwerden,
- Zahn- und Kieferschmerzen,
- Ulzerationen des Magen-Darmtraktes,
- Colitis ulcerosa und Morbus Crohn,
- hypertrophen Hautveränderungen,
- Psoriasis vulgaris und Neurodermitis,
- Warzen verschiedenster Genese,
- Cholecystitis, Cholelithiasis, Ikterus catarrhalis, Enteritiden, Ikterus infektiosus und latenten Hepatopathien, insbesondere Hepatitiden,
- dyspeptische Oberbauchbeschwerden, insbesondere bei funktionellen Beschwerden des ableitenden Gallensystems,
wobei die pflanzliche Zubereitung gemäss dem Verfahren nach einem der Ansprüche 1 bis 11 hergestellt ist, und
wobei die pflanzliche Zubereitung ein Teil- oder Vollextrakte aus Heil- und/oder Gewürzpflanzen oder Teilen davon ist, ausgewählt aus der Gruppe, bestehend aus
Berberis vulgaris L.
Peumus boldus Mol.
Sanguinaria canadensis L.
Chelidonium majus L.
Fumaria officinalis L.
Eschscholzia californica Cham.
Papaver somniferum L.
Papaver bracteatum Lindl.
Papaver rhoeas L..

19. Verwendung nach Anspruch 18, **dadurch gekennzeichnet, dass** das Medikament in einer pharmazeutisch akzeptablen Darreichungsform vorliegt, insbesondere in
- festen Darreichungsformen zur oralen Applikation, insbesondere in der Form einer Tablette, einer Filmtablette, eines Dragees, eines Pellets, einer Hartgelatine-Kapsel, einer Weichgelatine-Kapsel,
- flüssigen Darreichungsformen zur oralen, parenteralen, rektalen, vaginalen und topischen Applikation, insbesondere in der Form einer Tropflösung, eines Sprays, einer Injektionslösung, eines Sirups,
- halbfesten Darreichungsformen zur topischen, oralen, rektalen und vaginalen Applikation, insbesondere in der Form einer Crème, eines Gels, einer Salbe, eines Pflasters, einer Paste, eines Suppositoriums.

20. Verwendung nach einem der Ansprüche 18 bis 19, **dadurch gekennzeichnet, dass** die pflanzliche Zubereitung in Nano-Kapseln oder in Liposomen eingearbeitet ist.

21. Verwendung nach einem der Ansprüche 18 bis 20, **dadurch gekennzeichnet, dass** das Medikament noch wenigstens einen Zusatz- und/oder Hilfs- und/oder Wirkstoff enthält.

22. Verwendung nach einem der Ansprüche 18 bis 21, **dadurch gekennzeichnet, dass** die pflanzliche Zubereitung von 0,1 Gew.-% bis 10 Gew.-%, vorzugsweise von 1,0 Gew.-% bis 6,0 Gew.-%, Gesamtalkaloide enthält, bezogen auf die pflanzliche Zubereitung.

23. Pflanzliche Zubereitungen, enthaltend einen Teil- oder Vollextrakt aus der Pflanze Chelidonium majus L., insbesondere Herba Chelidonii, welche frei oder nahezu frei sind von unerwünschten, toxischen Alkaloiden, einschliesslich die Vorstufen und die Folgeprodukte dieser Verbindungen, wobei die unerwünschten, toxischen Alkaloide Sanguinarin und Chelerythrin sind, einschliesslich beliebige Gemische davon, dadurch erhältlich, dass man
a) die entsprechende Zubereitung auf einen Feststoffgehalt von 1 Gew.-% bis 20 Gew.-% durch die Hinzugabe von Wasser oder eines beliebigen Gemisches von Wasser und einem C₁- bis C₄-Alkohol, einem C₁- bis C₅-Keton, einem C₁- bis C₅-Aldehyd, einstellt, bezogen auf die so erhaltene Gesamtmischung, und mit einer solchen lipophilen Phase in Kontakt bringt oder vermischt, dass sich die zu entfernenden, unerwünschten Verbindungen in dieser lipophilen Phase lösen oder daran adsorbiert werden und sich hierin quantitativ oder nahezu quantitativ anreichern,
b) die erhaltene Mischung auf eine Temperatur im Bereich von -30°C bis +30°C bringt und dabei die unerwünschten Verbindungen von einem flüssigen Aggregatzustand in einen festen Aggregatzustand, unter Verwendung von Fetten, Wachsen oder Paraffinen, überführt,
c) die festen, unerwünschten Verbindungen mittels Membran-Separation abtrennt, und
d) die so gereinigte pflanzliche Zubereitung gewinnt.

## Claims

1. A process for the removal of undesired toxic alkaloids, including the precursors and the secondary products of these compounds, which are contained in plants or in vegetable preparations,
whereby these undesired compounds contain a quaternary nitrogen atom with a conjugated aromatic ring structure and/or conjugated double bonds, and are able for the adduct formation with other molecules, especially benzyl-, isoquinoline-, phenanthrene- and benzo-phenanthridine-alkaloids with these structure characteristics, including any mixtures thereof, with the exception of berberine and coptisine,
**characterized in that**
- in a first step the plant or the vegetable preparation, which contains said undesired compounds, is contacted or is mixed with such a lipophilic phase, that the undesired compounds to be removed are dissolved or are adsorbed in this lipophilic phase and are concentrated herein quantitatively or nearly quantitatively,
whereby in the case of a vegetable preparation the solids content is adjusted previously to an amount from 1 % by weight to 20 % by weight by the addition of water or any mixture of water and a C₁- to C₄-alcohol, a C₁- to C₅-ketone, a C₁- to C₅- aldehyde, referred to the so obtained total mixture,
- in a second step the lipophilic phase, which contains now the undesired compounds, is separated from the corresponding plant or from the corresponding vegetable preparation, and
- in a third step the so purified plant or the so purified vegetable preparation is obtained.

2. The process according to claim 1, **characterized in that** the precursors of the undesired compounds are dihydrosanguinarin and dihydrochelerythrin, including any mixtures thereof.

3. The process according to one of claims 1 to 2, **characterized in that** the toxic alkaloids are sanguinarin and chelerythrin, including any mixtures thereof.

4. The process according to one of claims 1 to 3, **characterized in that** the vegetable preparations are infusions, tinctures, fluids, spissum extracts, siccum extracts, dropping solutions, juices, tonics, injectable preparations.

5. The process according to one of claims 1 to 4, **characterized in that** the vegetable preparations are partial or complete extracts from medical and/or spice plants or parts thereof, especially selected from the group, consisting of
Berberis vulgaris L.
Peumus boldus Mol.
Sanguinaria canadensis L.
Chelidonium majus L.
Fumaria officinalis L.
Eschscholzia californica Cham.
Papaver somniferum L.
Papaver bracteatum Lindl.
Papaver rhoeas L..

6. The process according to one of claims 1 to 5, **characterized in that** that the lipophilic phase is of animal, vegetable, mineral or synthetic origin, and is especially not toxic, not easily inflammable, not explosive and not volatile, and is preferably selected from
- fats, such as cocoa butter, coconut fat;
- oils, such as neutral oils, sunflower oil, fractionated coconut oil, such as miglyol;
- waxes, such as stearins, yoyoba oil, beeswax, spermaceti, carnauba wax;
- paraffins, including vaseline;
- lipoids;
- sterols; and
- synthetic resins, especially polystyrene resins,
whereby all these compounds, as a single compound or as a mixture, preferably fulfill the requirements/definitions in the "Deutsches Arzneibuch", DAB, or in the British Pharmacopoe, BP, or according to the Food Chemical Codex, FCC, in the United States of America, or must correspond to these requirements/definitions, respectively.

7. The process according to one of claims 1 to 6, **characterized in that** in the first step the mixing of the components is realized at such a temperature, which lies between the freezing point and the boiling point of the respective mixture, whereby a temperature in the range from room temperature to 70°C is preferred.

8. The process according to one of claims 1 to 7, **characterized in that** in the first step the components are mixed together during about 1 hour, especially by means of shaking or stirring.

9. The process according to one of claims 1 to 8, **characterized in that** in the second step the separation is realized either by means of a phase separation of 2 liquid phases or by means of a phase separation of a liquid phase and a solid phase.

10. The process according to one of claims 1 to 9, **characterized in that** in the second step the separation of 2 liquid phases is realized by means of membrane-separation, whereby membranes with pore sizes in the range from 0.001 to 1.0 micrometer, especially from 0.1 to 0.3 micrometer, are preferred, especially glass-, metal-, ceramic- and synthetic membranes, for example made of polypropylene or Teflon.

11. The process according to one of claims 1 to 10, **characterized in that** in the case of a vegetable preparation
- in the second step the obtained mixture is brought to a temperature in the range from -30°C to +30°C and thereby the undesired compounds are transformed from a liquid state of aggregation into a solid state of aggregation,
- in the third step the solid, undesired compounds are separated, and
- in the fourth step the so purified vegetable preparation is obtained.

12. Vegetable preparations, which are free or nearly free from undesired toxic alkaloids, including the precursors and the secondary products of these compounds,
whereby the vegetable preparations are prepared according to the process according to one of claims 1 to 11, and
whereby the vegetable preparations are partial or complete extracts from medical and/or spice plants or parts thereof, selected from the group, consisting of
Berberis vulgaris L.
Peumus boldus Mol.
Sanguinaria canadensis L.
Chelidonium majus L.
Fumaria officinalis L.
Eschscholzia californica Cham.
Papaver somniferum L.
Papaver bracteatum Lindl.
Papaver rhoeas L..

13. Medicament, **characterized in that** it contains a vegetable preparation, which is free or nearly free from undesired toxic alkaloids, including the precursors and the secondary products of these compounds, and that it serves for the prevention, for the treatment or for the post-treatment of
- convulsive-like troubles in the area of the biliary ducts and of the gastrointestinal tract,
- menstruation troubles,
- toothache and jaw pains,
- ulcerations of the gastrointestinal tract,
- colitis ulcerosa and Crohn's disease,
- hypertrophic skin changes,
- psoriasis vulgaris and neurodermitis,
- warts of various genesis,
- cholecystitis, cholelithiasis, icterus catarrhalis, enteritides, icterus infectiosus and latent hepatopathies, especially hepatitides,
- dyspeptic troubles in the upper abdomen, especially on occasion of functional troubles of the derivate biliary system,
whereby the vegetable preparation is prepared according to the process according to one of claims 1 to 11, and
whereby the vegetable preparation is a partial or a complete extract from medical and/or spice plants or parts thereof, selected from the group, consisting of
Berberis vulgaris L.
Peumus boldus Mol.
Sanguinaria canadensis L.
Chelidonium majus L.
Fumaria officinalis L.
Eschscholzia californica Cham.
Papaver somniferum L.
Papaver bracteatum Lindl.
Papaver rhoeas L..

14. Medicament according to claim 13, **characterized in that** it is in a pharmaceutically acceptable administrative form, especially in
- solid administrative forms for oral application, especially in the form of a tablet, a film tablet, a dragee, a pellet, a hard gelatin-capsule, a soft gelatin-capsule,
- liquid administrative forms for oral, parenteral, rectal, vaginal and topic application, especially in the form of a dropping solution, a spray, an injection solution, a syrup,
- semisolid administrative forms for topic, oral, rectal and vaginal application, especially in the form of a cream, a gel, an ointment, a plaster, a paste, a suppository.

15. Medicament according to one of claims 13 to 14, **characterized in that** the vegetable preparation is incorporated into nano-capsules or into liposomes.

16. Medicament according to one of claims 13 to 15, **characterized in that** it contains additionally at least one additive and/or auxiliary agent and/or active compound.

17. Medicament according to one of claims 13 to 16, **characterized in that** the vegetable preparation contains from 0.1 % by weight to 10 % by weight, preferably from 1.0 % by weight to 6.0 % by weight, of total alkaloids, referred to the vegetable preparation.

18. Use of a vegetable preparation, which is free or nearly free from undesired toxic alkaloids, including the precursors and the secondary products of these compounds, for the preparation of a medicament for the prevention, for the treatment or for the post-treatment of
- convulsive-like troubles in the area of the biliary ducts and of the gastrointestinal tract,
- menstruation troubles,
- toothache and jaw pains,
- ulcerations of the gastrointestinal tract,
- colitis ulcerosa and Crohn's disease,
- hypertrophic skin changes,
- psoriasis vulgaris and neurodermitis,
- warts of various genesis,
- cholecystitis, cholelithiasis, icterus catarrhalis, enteritides, icterus infectiosus and latent hepatopathies, especially hepatitides,
- dyspeptic troubles in the upper abdomen, especially on occasion of functional troubles of the derivate biliary system,
whereby the vegetable preparation is prepared according to the process according to one of claims 1 to 11, and
whereby the vegetable preparation is a partial or a complete extract from medical and/or spice plants or parts thereof, selected from the group, consisting of
Berberis vulgaris L.
Peumus boldus Mol.
Sanguinaria canadensis L.
Chelidonium majus L.
Fumaria officinalis L.
Eschscholzia californica Cham.
Papaver somniferum L.
Papaver bracteatum Lindl.
Papaver rhoeas L..

19. Use according to claim 18, **characterized in that** the medicament is in a pharmaceutically acceptable administrative form, especially in
- solid administrative forms for oral application, especially in the form of a tablet, a film tablet, a dragee, a pellet, a hard gelatin-capsule, a soft gelatin-capsule,
- liquid administrative forms for oral, parenteral, rectal, vaginal and topic application, especially in the form of a dropping solution, a spray, an injection solution, a syrup,
- semisolid administrative forms for topic, oral, rectal and vaginal application, especially in the form of a cream, a gel, an ointment, a plaster, a paste, a suppository.

20. Use according to one of claims 18 to 19, **characterized in that** the vegetable preparation is incorporated into nano-capsules or into liposomes.

21. Use according to one of claims 18 to 20, **characterized in that** the medicament contains additionally at least one additive and/or auxiliary agent and/or active compound.

22. Use according to one of claims 18 to 21, **characterized in that** the vegetable preparation contains from 0.1 % by weight to 10 % by weight, preferably from 1.0 % by weight to 6.0 % by weight, of total alkaloids, referred to the vegetable preparation.

23. Vegetable preparations, containing a partial or complete extract from the plant Chelidonium majus L., especially Herba Chelidonii, which are free or nearly free of undesired toxic alkaloids, including the precursors and the secondary products of these compounds, whereby the undesired toxic alkaloids are sanguinarin and chelerythrin, including any mixtures thereof,
obtainable in that
a) the corresponding preparation is adjusted to a solids content from 1 % by weight to 20 % by weight by the addition of water or any mixture of water and a C₁- to C₄-alcohol, a C₁- to C₅-ketone, a C₁- to C₅- aldehyde, referred to the so obtained total mixture, and is contacted or is mixed with such a lipophilic phase, that the undesired compounds to be removed are dissolved or are adsorbed in this lipophilic phase and are concentrated herein quantitatively or nearly quantitatively,
b) the so obtained mixture is brought to a temperature in the range from -30°C to +30°C and thereby the undesired compounds are transformed from a liquid state of aggregation into a solid state of aggregation, using fats, waxes or paraffins,
c) the solid undesired compounds are separated by means of membrane separation, and
d) the so purified vegetable preparation is obtained.

## Revendications

1. Procédé permettant d'éliminer des plantes ou des préparations végétales les alcaloïdes toxiques indésirables, y compris les précurseurs et les dérivés de ces composés, ces composés indésirables contenant un atome d'azote quaternaire avec une structure cyclique aromatique conjuguée et/ou des doubles liaisons conjuguées et étant aptes à former des adduits avec d'autres molécules, en particulier les alcaloïdes benzyliques, isoquinoléiniques, phénanthréniques et benzophénanthridiniques possédant ces caractéristiques structurelles, y compris des mélanges quelconques de ceux-ci, à l'exception de la berbérine et de la coptisine, **caractérisé en ce que**
- dans une première étape, on met en contact ou on mélange la plante ou la préparation à base végétale qui renferme les composés indésirables cités, avec une phase lipophile telle que les composés indésirables à éliminer se dissolvent dans cette phase lipophile ou sont adsorbés sur celle-ci et s'y concentrent de manière quantitative ou quasi quantitative,
dans le cas d'une préparation à base végétale, la teneur en matières solides de celle-ci étant ajustée au préalable entre 1 et 20 % en poids par adjonction d'eau ou d'un mélange quelconque d'eau et d'un alcool en C₁-C₄, d'une cétone en C₁-C₅ ou d'un aldéhyde en C₁-C₅, par rapport au mélange total ainsi obtenu,
- dans une deuxième étape, on sépare la phase lipophile, qui renferme à présent les composés indésirables, de la plante ou de la préparation à base végétale respective, et
- dans une troisième étape, on récolte la plante ou la préparation à base végétale ainsi purifiée.

2. Procédé selon la revendication 1, **caractérisée en ce que** les précurseurs des composés indésirables sont la dihydrosanguinarine et la dihydrochélérythrine, y compris leurs mélanges quelconques.

3. Procédé selon une des revendications 1 à 2, **caractérisé en ce que** les alcaloïdes toxiques sont la sanguinarine et la chélérythrine, y compris leurs mélanges quelconques.

4. Procédé selon une des revendications 1 à 3, **caractérisé en ce que** les préparations à base végétale sont des infusions, des teintures, des liquides, des extraits épais, des extraits secs, des solutions-gouttes, des sucs, des toniques et des préparations injectables.

5. Procédé selon une des revendications 1 à 4, **caractérisé en ce que** les préparations à base végétale sont des extraits partiels ou complets de plantes médicinales et/ou aromatiques ou de parties de celles-ci, sélectionnées en particulier dans le groupe constitué de
Berberis vulgaris L.
Peumus boldus Mol.
Sanguinaria canadensis L.
Chelidonium majus L.
Fumaria officinalis L.
Eschscholzia californica Cham.
Papaver somniferum L.
Papaver bracteatum Lindl.
Papaver rhoeas L..

6. Procédé selon une des revendications 1 à 5 **caractérisé en ce que** la phase lipophile est d'origine animale, végétale, minérale ou synthétique et, en particulier, n'est pas toxique, pas facilement inflammable, pas explosive et pas volatile, et est sélectionnée de préférence parmi
- des graisses, telles le beurre de cacao ou la graisse de coco;
- des huiles, telles des huiles neutres, l'huile de tournesol, l'huile de noix de coco fractionnée, telle le myglyol;
- des cires, telles la stéarine, l'huile de jojoba, la cire d'abeilles, la cétine et la cire de camauba;
- des paraffines, y compris la vaseline;
- des lipoïdes;
- des stérines; et
- des résines synthétiques, en particulier des résines polystyréniques,
tous ces composés devant, individuellement ou en mélange, répondre ou satisfaire de préférence aux exigences/définitions respectivement du *Deutsches Arzneibuch*, DAB, de la pharmacopée britannique, BP, ou du Food Chemical Codex, FCC, aux États-Unis.

7. Procédé selon une des revendications 1 à 6, **caractérisé en ce que** dans la première étape, le mélange des composants se déroule à une température qui est comprise entre le point de congélation et le point d'ébullition du mélange respectif, une température comprise dans la plage s'étendant de la température ambiante à 70 °C étant préférée.

8. Procédé selon une des revendications 1 à 7, **caractérisé en ce que** dans la première étape, on mélange les composants ensemble pendant environ 1 heure, en particulier en les secouant ou en les agitant.

9. Procédé selon une des revendications 1 à 8, **caractérisé en ce que** dans la deuxième étape, la séparation se déroule soit à l'aide d'une séparation de phase de deux phases liquides ou d'une séparation de phase d'une phase liquide et d'une phase solide.

10. Procédé selon une des revendications 1 à 9, **caractérisé en ce que** dans la deuxième étape, la séparation de deux phases liquides se déroule par une séparation membranaire, des membranes présentant des ouvertures de pore situées dans l'intervalle de 0,001 à 1,0 µm, en particulier de 0,1 à 0,3 µm étant préférées, en particulier des membranes en verre, métal, céramique et matière synthétique, par exemple en polypropylène ou en téflon.

11. Procédé selon une des revendications 1 à 10, **caractérisé en ce que** dans le cas de préparations à base végétale,
- dans la deuxième étape, on ajuste la température du mélange obtenu dans l'intervalle de - 30 à + 30 °C et on fait ainsi passer les composés indésirables d'un état d'agrégat liquide dans un état d'agrégat solide,
- dans la troisième étape, on sépare les composés indésirables solides, et
- dans la quatrième étape, on récolte la préparation à base végétale ainsi purifiée.

12. Préparations à base végétale, qui sont exemptes ou quasi exemptes d'alcaloïdes toxiques indésirables, y compris les précurseurs et les dérivés de ces composés,
les préparations à base végétale étant préparées en appliquant le procédé selon une des revendications 1 à 11, et
les préparations à base végétale étant des extraits partiels ou complets de plantes médicinales et/ou aromatiques ou de parties de celles-ci, et étant sélectionnées dans le groupe constitué de
Berberis vulgaris L.
Peumus boldus Mol.
Sanguinaria canadensis L.
Chelidonium majus L.
Fumaria officinalis L.
Eschscholzia califomica Cham.
Papaver somniferum L.
Papaver bracteatum Lindl.
Papaver rhoeas L..

13. Médicament, **caractérisé en ce qu'**il renferme une préparation à base végétale, qui est exempte ou quasi exempte d'alcaloïdes toxiques indésirables, y compris les précurseurs et les dérivés de ces composés, et est destiné à la prévention, au traitement ou au traitement postérieur des
- douleurs spasmodiques dans la région des voies biliaires et du tube digestif,
- douleurs menstruelles,
- douleurs maxillo-dentaires,
- ulcérations du tube digestif,
- colite ulcéreuse et maladie de Crohn,
- altérations hypertrophiques de la peau,
- psoriasis vulgaire et neurodermite,
- verrues d'étiologies diverses,
- cholécystite, cholélithiase, hépatite virale, entérite, ictère infectieux et hépatopathies latentes, en particulier hépatites,
- troubles gastriques dyspepsiques, en particulier en cas de trouble fonctionnel de l'appareil biliaire excréteur,
la préparation à base végétale étant préparée selon le procédé conforme à l'une des revendications 1 à 11, et
la préparation à base végétale étant un extrait partiel ou complet de plantes médicinales et/ou aromatiques ou de parties de celles-ci, sélectionnées dans le groupe constitué de
Berberis vulgaris L.
Peumus boldus Mol.
Sanguinaria canadensis L.
Chelidonium majus L.
Fumaria officinalis L.
Eschscholzia californica Cham.
Papaver somniferum L.
Papaver bracteatum Lindl.
Papaver rhoeas L..

14. Médicament selon la revendication 13, **caractérisé en ce qu'**il est sous une présentation acceptable du point de vue pharmaceutique, en particulier sous
- des présentations solides destinées à l'administration orale, en particulier sous la forme de comprimé, de comprimé enrobé, de dragée, de pellet, de capsule en gélatine dure ou de capsule en gélatine molle,
- des présentations liquides destinées à l'administration orale, parentérale, rectale, vaginale et topique, en particulier sous la forme de solution-gouttes, d'aérosol, de solution injectable ou de sirop,
- des présentations semi-solides destinées à l'administration topique, orale, rectale et vaginale, en particulier sous la forme de crème, de gel, de pommade, d'emplâtre, de pâte ou de suppositoire.

15. Médicament selon une des revendications 13 à 14, **caractérisé en ce que** la préparation à base végétale est incorporée dans des nanocapsules ou des liposomes.

16. Médicament selon une des revendications 13 à 15, **caractérisé en ce qu'**il renferme encore au moins un additif et/ou un adjuvant et/ou un principe actif.

17. Médicament selon une des revendications 13 à 16, **caractérisé en ce que** la préparation à base végétale contient de 0,1 à 10 % en poids, de préférence de 1,0 à 6,0 % en poids d'alcaloïdes totaux par rapport à la préparation à base végétale.

18. Utilisation d'une préparation à base végétale, qui est exempte ou quasi exempte d'alcaloïdes toxiques indésirables, y compris les précurseurs et les dérivés de ces composés, pour préparer un médicament destiné à la prévention, au traitement ou au traitement postérieur des
- douleurs spasmodiques dans la région des voies biliaires et du tube digestif,
- douleurs menstruelles,
- douleurs maxillo-dentaires,
- ulcérations du tube digestif,
- colite ulcéreuse et maladie de Crohn,
- altérations hypertrophiques de la peau,
- psoriasis vulgaire et neurodermite,
- verrues d'étiologies diverses,
- cholécystite, cholélithiase, hépatite virale, entérite, ictère infectieux et hépatopathies latentes, en particulier hépatites,
- troubles gastriques dyspepsiques, en particulier en cas de trouble fonctionnel de l'appareil biliaire excréteur,
la préparation à base végétale étant préparée selon le procédé conforme à l'une des revendications 1 à 11, et
la préparation à base végétale étant un extrait partiel ou complet de plantes médicinales et/ou aromatiques ou de parties de celles-ci, sélectionnées dans le groupe constitué de
Berberis vulgaris L.
Peumus boldus Mol.
Sanguinaria canadensis L.
Chelidonium majus L.
Fumaria officinalis L.
Eschscholzia califomica Cham.
Papaver somniferum L.
Papaver bracteatum Lindl.
Papaver rhoeas L..

19. Utilisation selon la revendication 18, **caractérisée en ce que** le médicament est sous une forme d'administration acceptable du point de vue pharmaceutique, en particulier sous
- des présentations solides destinées à l'administration orale, en particulier sous la forme de comprimé, de comprimé enrobé, de dragée, de pellet, de capsule en gélatine dure ou de capsule en gélatine molle,
- des présentations liquides destinées à l'administration orale, parentérale, rectale, vaginale et topique, en particulier sous la forme de solution-gouttes, d'aérosol, de solution injectable ou de sirop,
- des présentations semi-solides destinées à l'administration topique, orale, rectale et vaginale, en particulier sous la forme de crème, de gel, de pommade, d'emplâtre, de pâte ou de suppositoire.

20. Utilisation selon une des revendications 18 à 19, **caractérisée en ce que** la préparation à base végétale est incorporée dans des nanocapsules ou des liposomes.

21. Utilisation selon une des revendications 18 à 20, **caractérisée en ce que** le médicament renferme encore au moins un additif et/ou un adjuvant et/ou un principe actif.

22. Utilisation selon une des revendications 18 à 21, **caractérisée en ce que** la préparation à base végétale contient de 0,1 à 10 % en poids, de préférence de 1,0 à 6,0 % en poids d'alcaloïdes totaux par rapport à la préparation à base végétale.

23. Préparations à base végétale contenant un extrait partiel ou complet de la plante Chelidonium majus L., en particulier Herba Chelidonii, qui sont exemptes ou quasi exemptes d'alcaloïdes toxiques indésirables, y compris les précurseurs et les dérivés de ces composés, les alcaloïdes toxiques indésirables étant la sanguinarine et la chélérythrine, y compris des mélanges quelconques de ceux-ci, pouvant être obtenues
a) en ajustant la teneur en matières solides de la préparation correspondante dans l'intervalle de 1 à 20 % en poids, par adjonction d'eau ou d'un mélange quelconque d'eau et d'un alcool en C₁ à C₄, d'une cétone en C₁-C₅ ou d'un aldéhyde en C₁-C₅, par rapport au mélange total ainsi obtenu et en la mettant en contact ou en la mélangeant avec une phase lipophile telle que les composés indésirables à éliminer se dissolvent dans cette phase lipophile ou sont adsorbées sur celle-ci et se concentrent dans ou sur celle-ci quantitativement ou quasi quantitativement,
b) en ajustant la température du mélange obtenu dans l'intervalle de - 30 à + 30 °C, et en faisant ainsi passer les composés indésirables d'un état d'agrégat liquide dans un état d'agrégat solide, en utilisant des graisses, des cires ou des paraffines,
c) en séparant les composés indésirables solides au moyen d'une séparation membranaire, et
d) en récoltant la préparation à base végétale ainsi purifiée.
